(19) Europäisches Patentamt
European Patent Office
Office européen des brevets

(11) Veröffentlichungsnummer: **0 082 804**
**B1**

(12) # EUROPÄISCHE PATENTSCHRIFT

(45) Veröffentlichungstag der Patentschrift: **12.08.87**

(51) Int. Cl.⁴: **C 07 D 317/28**, C 07 D 317/34, C 07 C 103/78, C 07 C 103/46, A 61 K 49/04

(21) Anmeldenummer: **82730147.4**

(22) Anmeldetag: **16.12.82**

(54) **2-Amino-1-(1,3-dioxolan-4-yl)-ethanol-Verbindungen, deren Herstellung und Verwendung zur Weiterverarbeitung.**

(30) Priorität: **18.12.81 DE 3150917**

(43) Veröffentlichungstag der Anmeldung: **29.06.83 Patentblatt 83/26**

(45) Bekanntmachung des Hinweises auf die Patenterteilung: **12.08.87 Patentblatt 87/33**

(84) Benannte Vertragsstaaten: **AT BE CH DE FR GB IT LI LU NL SE**

(56) Entgegenhaltungen:
**EP-A-0 022 744**
**EP-A-0 026 281**
**EP-A-0 033 426**
**BE-A-836 355**
**DE-A-2 031 724**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Niedballa, Ulrich, Dr., Gosslerstrasse 28a, D-1000 Berlin 33 (DE)**
Erfinder: **Gries, Heinz, Dr., Helmstedter Strasse 19, D-1000 Berlin 31 (DE)**

**EP 0 082 804 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft 2-Amino-1-(1,3-dioxolan-4-yl)-ethanol-Verbindungen der allgemeinen Formel I

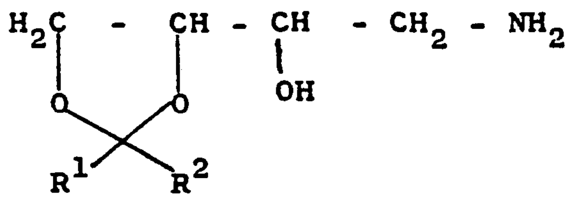

(I),

worin $R^1$ und $R^2$ gleich oder verschieden sind und Wasserstoff, eine niedere Alkylgruppe mit 1 bis 4 Kohlenstoffatomen darstellt oder, wenn $R^1$ eine Alkoxygruppe mit 1 bis 4 Kohlenstoffatomen bedeutet, $R^2$ eine Alkylgruppe mit 1 bis 4 Kohlenstoffatomen darstellt, oder $R^1$ und $R^2$ zusammen einen Alkylenrest mit 5 oder 6 Kohlenstoffatomen bedeuten.

Kleine, hochsubstituierte hydrophile Amine werden häufig in der Technik verwendet z. B. als Hilfsstoffe in der Textil-, Leder- und Papierindustrie, als Rohstoffe für Detergenzien, als lösungsvermittelnde Substituenten bei Pharmazeutika. Von grösster Wichtigkeit ist ihre Verwendung bei der Synthese von Röntgenkontrastmitteln. Dabei wird ein Grundkörper, der zum Zwecke der Absorption von Röntgenstrahlen mit Jodatomen hochsubstituiert ist, mit hydrophilen Resten substituiert, um Wasserlöslichkeit und physiologische Verträglichkeit zu gewährleisten, wobei diese Substituenten durch ihre Struktur diese Eigenschaften der Röntgenkontrastmittel entscheidend beeinflussen.

Die Synthese eines neuen Röntgenkontrastmittels ist deshalb häufig von der Verfügbarkeit eines neuen geeigneten Zwischenproduktes abhängig, das als Substituent dem Grundkörper die gewünschten Eigenschaften verleiht.

Da die meisten Techniken der Röntgendiagnose den Einsatz grösserer Mengen Kontrastmittel erfordern, ist es zudem notwendig, das Röntgenkontrastmittel und seine Zwischenstufen bequem und wirtschaftlich darzustellen.

Als besonders vorteilhaft erweist es sich, die Verknüpfung zwischen dem jodhaltigen Grundkörper und dem hydrophilen Substituenten über eine Amidbindung vorzunehmen: die Bindung ist stabil und trägt aufgrund ihrer Polarität ebenfalls zur Wasserlöslichkeit des Gesamtmoleküls bei.

In der Literatur gibt es viele Beispiele für diesen Verbindungstyp, wobei die Acylfunktion auch mehrfach enthalten sein kann. So beschreibt das Belgische Patent Nr. 836 355 die Synthese des 5-(α-Hydroxypropionylamino)-2,4,6-triiodisophthalsäure-N,N'-bis-(1,3-dihydroxyprop-2-yl)-diamids (Iopamidol), während die Synthese von Metrizamid [2-(3-Acetamido-5-N-methylacetamido-2,4,6-triiod-benzamido)-2-desoxy-D-glucose] in DOS 2 031 724 beschrieben ist. Beide Verbindungen sind bereits im Handel.

In der europäischen Anmeldung 0 033 426 werden das 5-Acet-amido-2,4,6-triiodisophthalsäure-N,N'-bis(1,3,4-trihy-droxybut-2-yl)-diamid sowie das 2,4,6-Triiodtrimesinsäure-N,N',N''-tris(1,3,4-trihydroxybut-2-yl)-triamid beschrieben, die beide 2-Amino-1,3,4-butantriol als Aminkomponente enthalten.

Bei den dort beschriebenen Reaktionen werden jodierte Grundkörper verwendet, die eine oder mehrere Säurechloridgruppen tragen, wobei diese mit einem durch Hydroxygruppen substituierten Alkylamin umgesetzt werden.

Die bei einem Röntgenkontrastmittel als Endprodukt erwünschte hohe Hydrophilität erweist sich jedoch als Nachteil bei der Herstellung der Verbindungen, da solche Verbindungen durch die geringe Neigung zur Kristallisation sowie die schlechte Löslichkeit in aprotischen, niedrig biedenden Lösungsmitteln schwer zu reinigen sind.

Die Reinheit der Endprodukte und damit auch der Aufwand zur Endreinigung wird entscheidend beeinflusst durch die Reaktionsgeschwindigkeit mit der Säurechlorid und Amin reagieren und die bei einer Aminomethylgruppe $-CH_2-NH_2$ grösser ist als bei einem sterisch gehinderten Amin. Deshalb erweisen sich für die Synthese von Röntgenkontrastmitteln solche Amine als am geeignetsten, bei denen die Aminogruppe nicht sterisch gehindert und die Hydroxylgruppen ganz oder teilweise geschützt sind.

Verwendet man beispielsweise Amino-trihydroxybutan als hydroxyliertes Alkylamin, von dem 4 isomere Verbindungen existieren

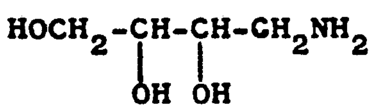

(Va),

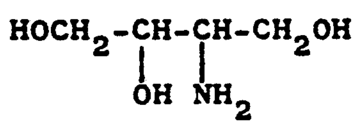

(Vb),

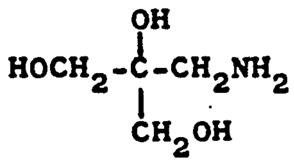

(Vc),

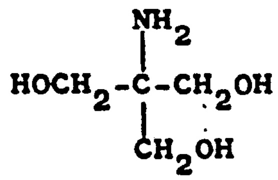

(Vd),

wird Verbindung Va für die Reaktion mit einem Säurechlorid am günstigsten sein, da die Aminogruppe am wenigsten sterisch gehindert ist. Doch da die noch vorhandene primäre Hydroxylgruppe ebenfalls mit dem Säurechlorid in Reaktion treten und zu Nebenprodukten führen kann, ist es vorteilhaft, Verbindung Va als Hydroxylgruppen-geschütztes Zwischenprodukt der allgemeinen Formel I zu verwenden.

Änliche Amine werden auch in der europäischen Patentanmeldung 0 033 426 verwendet: wie beispielsweise Verbindungen der Formel VI

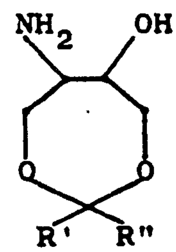

(VI),

Sie haben jedoch den entscheidenden Nachteil, dass sie eine sterisch gehinderte Aminogruppe

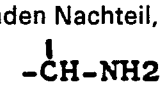

enthalten, die drastischere Reaktionsbedingungen erfordert als eine Aminomethylengruppe $-CH_2-NH_2$ und so zu Nebenprodukten führen, die die Reinigung der Endprodukte erschweren. Dazu kommt, dass die Verbindungen der Formel VI als Dioxepane ein sterisch gespanntes 7-Ring-System darstellen, das erfahrungsgemäss gegenüber schwach saurem Medium weniger beständig ist als ein 5-Ring-System, und durch vorzeitige Schutzgruppenspaltung die Gefahr des Auftretens von Nebenprodukten erhöhen.

Diese Nachteile sind bei den erfindungsgemässen Verbindungen der allgemeinen Formel I weitgehend beseitigt.

Die erfindungsgemässen Amine der Formel I besitzen eine reaktionsfähige, kaum gehinderte Aminogruppe $-CH_2-NH_2$, die kürzere Reaktionszeiten bei der Acylierung ermöglicht, was besonders für die Umsetzung empfindlicher Verbindungen wichtig ist, da weniger Nebenprodukte erhalten werden. Die im Molekül von Verbindungen der allgemeinen Formel I vorhandene Schutzgruppe für die 3,4-stän-digen Hydroxygruppen ist genügend stabil, um ohne vorzeitige Spaltung die Reaktionsbedingungen für die Weiterverarbeitung zu jodhaltigen Röntgenkontrastmitteln zu ermöglichen. Sie sind aber mit Methoden, die dem Fachmann bekannt sind, beispielsweise durch verdünnte wässrige Mineralsäure unter leichtem Erwärmen oder bei Raumtemperatur, leicht spaltbar, wenn es erforderlich ist.

Die erfindundsgemässen Amine der allgemeinen Formel I eignen sich hervorragend zur Herstellung der schattengebenden Verbindungen von Röntgenkontrastmitteln durch Umsetzung eines jodhältigen Säurechlorids oder Bis-säurechlorids zum jodhaltigen Monoamid bzw. Bis-amid. So kann man beispielsweise 5-[-N-(2-Hydroxyäthyl)-acetylamino]-2,4,6-trijod-isophthalsäure-bis-(2,3,4-trihydroxy-but-1-yl)-diamid, eine schattengebende Verbindung mit ausgezeichneter Verträglichkeit und Wasser-Löslichkeit, durch Verwendung eines Amins der Formel I nach folgender Arbeitsvorschrift herstellen:

1. A) <u>5-Acetylamino-2,4,6-trijod-isophthalsäure-bis-(2,3,4-trihydroxy-but-1-yl)-diamid</u>

Zu einer Lösung oder Suspension von 180 mMol 2-Amino-1-(2,2-dimethyl-1,3-dioxolan-4-yl)-ethanol in 100 ml Dimethylacetamid tropft man innerhalb von 15 Minuten unter schwacher Kühlung und Rühren bei Raumtemperatur eine Lösung von 51 g (80 mMol) 5-Acetylamino-2,4,6-trijod-isophthalsäuredichlorid in 100 ml Dimethylacetamid zu. Anschliessend gibt man 25,1 ml (180 mMol) Triäthylamin tropfenweise zu. Nach Rühren über Nacht wird die Suspension 4 Stunden auf 50°C erwärmt, danach abgekühlt und mit 4,5 ml wässriger konz. Salzsäure angesäuert. Nach einigen Stunden wird das ausgefallene Triäthylamin-Hydrochlorid (ca. 22 g, 90 % der Theorie) abgesaugt und das Filtrat im Vakuum weitgehend eingeengt. Dieser Rückstand wird mit 200 ml Wasser und 4 ml wässriger konz. Natronlauge versetzt (ca. pH = 10) und einige Stunden gerührt. Bei dieser

wässrig-sauren und wässrig-alkalischen Behandlung werden in den Amidresten vorliegende Schutzgruppen im allgemein quantitativ abgespalten, was durch Dünnschichtchromatographie geprüft wird. Anderenfalls werden durch wiederholte wässrig-saure Behandlung die Schutzgruppen abgespalten. Die erhaltene Lösung wird mit 500 ml Kationenaustauscher IR 120 behandelt und das Eluat im Vakuum eingeengt. Nach Führen mit Wasser und Kochen mit Ethanol werden 47,8 g (74 % der Theorie) 5-Acetylamino-2,4,6-trijod-isophthal-säure-bis-(2,3,4-trihydroxy-but-1-yl)-diamid erhalten.

Fp.: 279-283° C (Zersetzung)

Jod, ber. 47,17 %, gef. 47,32 %.

Wie oben bereits erwähnt, gestatten die erfindungsgemäßen Verbindungen der Formel I, falls erwünscht, die Isolierung des Umsetzungsproduktes von 1 mit dem jeweiligen Säurechlorid vor der Abspaltung des Hydroxygruppenschutzes, der dafür genügend stabil ist. Da diese Zwischenprodukte mit noch erhaltenen Hydroxylgruppenschutz eine niedrige Polarität besitzen als die daraus durch Schutzgruppenspaltung entstehenden Polyhydroxyverbindungen, lassen sich diese Verbindungen leichter isolieren und durch Auswaschen, Umkristallisieren oder Chromatographieren reinigen.

Die im Anschluß an die Isolierung oder Reinigung erfolgende Schutzgruppenabspaltung liefert dann die Polyhydroxyverbindung. Die nachfolgende Arbeitsvorschrift soll das verdeutlichen:

1 B) 5-Acetylamino-2,4,6-trijod-isophthalsäure-bis-[2-hydroxy-2-(2,2-dimethyl-1,3-dioxolan-4-yl)-äthyl]-diamid

Wie unter 1 A) beschrieben, setzt man 180 mMol 2-Amino-1-(2,2-dimethyl-1,3-dioxolan-4-yl)-ethanol mit 51 g (80 mMol) 5-Acetylamino-2,4,6-trijod-isophthalsäure-dichlorid um und arbeitet das Reaktionsgemisch vor der Zugabe von 4,5 ml wässriger konzentrierter Salzsäure nach folgender Vorschrift auf:

Die entstandene Suspension wird abfiltriert, das Filtrat im Vakuum zur Trockne eingedampft, der Eindampfrückstand mit Aceton, danach mit Methylenchlorid und schließlich mit Wasser, dem Ammoniak bis pH = 9 zugesetzt wurde, gründlich ausgerührt.

Man erhält 38,5 g (54,2 % d.Th.) 5-Acetylamino-2,4,6-trijod-isophthalsäure-bis-[2-hydroxy-2-(2,2-dimethyl-1,3-dioxalan-4-yl)-äthyldiamid; Fp.: 277-278° C.

Führt man mit dieser Verbindung die in der Vorschrift 1 A beschriebene saure Behandlung mit konzentrierter Salzsäure und die daran anschließende Aufarbeitung durch, erhält man das unter 1 A beschriebene 5-Acetylamino-2,4,6-trijod-isophthalsäure-bis-(2,3,4-trihydroxy-but-1-yl)-diamid.

2. 5-[N-(2-Hydroxyäthyl)-acetylamino]-2,4,6-trijod-isophthalsäure-bis-(2,3,4-trihydroxy-but-1-yl)-diamid:

Eine Methylatlösung aus 100 ml Methanol und 2,48 g (108 mMol) Natrium wird mit 110 ml 1,2-propylengly-kol, 40,3 g (50 mMol) 5-Acetylamino-2,4,6-trijod-isophthalsäure-bis-(2,3,4-trihydroxy-but-1-yl)-diamid und zum Nachspülen mit 50 ml Methanol versetzt. Beim Rühren und Erwärmen auf 50° C entsteht eine Lösung, aus der im Vakuum das Methanol abdestilliert wird. Dann wird die Lösung mit 6,7 ml (100 mMol) 2-Chloräthanol versetzt und 5 Stunden bei 50° C weitergerührt. Nach dem Abkühlen wird die Suspension mit einem Liter Aceton versetzt und nach einer Stunde filtriert. Der natriumchloridhaltige Niederschlag wird erneut mit Aceton ausgerührt und abgesaugt. Dieses Gemisch (ca. 47 g) wird in 470 ml Wasser gelöst und über eine Säule mit 600 ml Kationenaustauscher IR 120 gegeben. Das wässrige Eluat wird im Vakuum weitgehend eingeengt, der Rückstand mit 370 ml Wasser aufgenommen und analog mit dem Anionenaustauscher IRA 410 behandelt und aufgearbeitet. Das Auskochen des Rückstandes mit 165 ml Isopropanol liefert 27,6 g (64,7 % der Theorie).

Titelverbindung, Fp.: 283 - 287° C (Zersetzung).

Jod ber. 44,73 % gef. 44,2 %.

Die Erfindung betrifft auch ein Verfahren zur Herstellung von 2-Amino-1-(1, 3-dioxolan-4-yl)-ethanol-Verbindungen der allgemeinen Formel I gemäss Anspruch 1, dadurch gekennzeichnet, dass man in an sich bekannter Weise

a) ein 1,3-Dioxolanepoxid der allgemeinen Formel

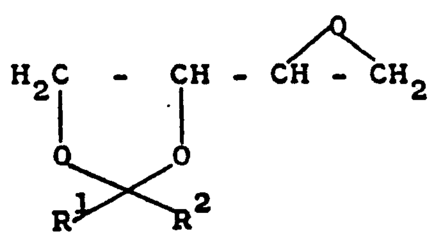

(II),

worin $R^1$ und $R^2$ die im Anspruch 1 angegebenen Bedeutungen besitzen, mit $NH_3$ umsetzt,

oder

b) eine Azid-Verbindung der allgemeinen Formel III

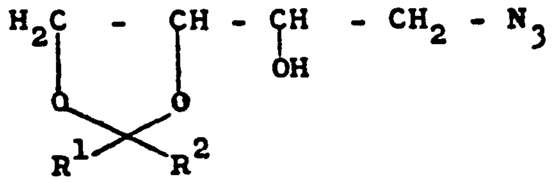

(III),

worin $R^1$ und $R^2$ die im Anspruch 1 angegebenen Bedeutungen besitzen, hydriert.

Zur Herstellung der erfindungsgemässen Verbindungen der Formel I nach Verfahrensvariante a) wird die entsprechende 1,2-Oxidoverbindung der allgemeinen Formel II in einem geeigneten polaren Lösungsmittel wie Wasser, Dimethylformamid o.ä. mit überschüssigem Ammoniak bei einer Temperatur von 80° bis 140° vorzugsweise 100°C bis 130°C in einem Druckgefäss bis zur vollständigen Öffnung des Oxidoringes erhitzt, aufgearbeitet und durch Kristallisation gereinigt.

Die für diese Reaktion erforderliche 1,2-Oxidoverbin-dung der allgemeinen Formel II erhält man nach Methoden, die dem Fachmann bekannt sind, beispielsweise nach folgendem Schema:

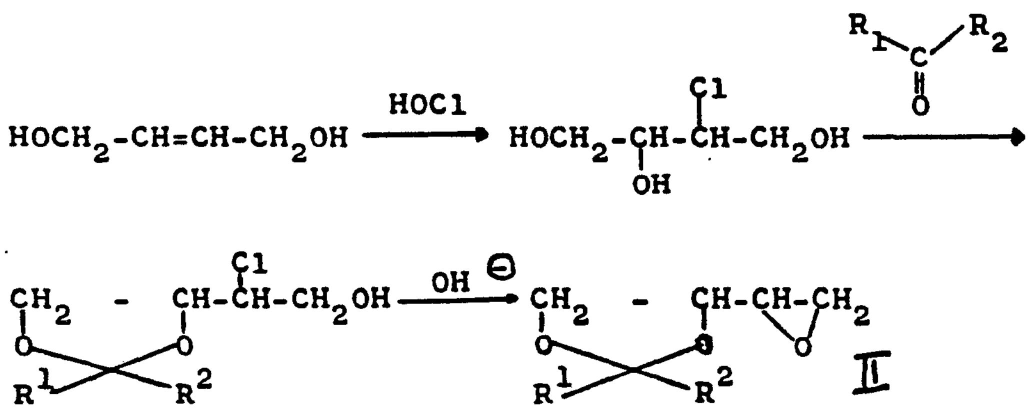


II

($R^1$ und $R^2$ haben die in Anspruch 1 angegebene Bedeutung).

Am Beispiel der Herstellung von 2-(2,2-Dimethyl-1,3-dioxolan-4-yl)-ethylenoxid (Verbindung der allgemeinen Formel II mit $R^1 = R^2 = CH_3$)soll das Verfahren nähen erläutert werden:

1. (W.Reppe et al., Liebigs Am.Chem. 596 137 [1955]). In eine Lösung aus 182 g cis-1,4-Dihydroxy-2-buten und 288 g kristallisierter Soda ($Na_2CO_3$ x 10 $H_2O$) werden unter starkem Rühren und Kühlung auf 0 - 5°C innerhalb von 6 Stunden 150 g Chlor eingeleitet. Es wird noch 1 Stunde nachgerührt, im Vakuum bei 50-55°C eingeengt und mit 0,6 l Ethanol verdünnt. Nach Stehen über Nacht wird vom Natriumchlorid abfiltriert und mit Ethanol gut nachgewaschen. Lösung und Filtrat werden eingeengt.

Das 3-Chlor-1,2,4-butantriol wird als gelbes Öl erhalten.

Ausbeute: 272 g (97 % der Theorie) 3-Chlor-1,2,4-Butantriol.

2. Zu einer gerührten und mit Wasser gekühlten Lösung von 272 g 3-Chlor-1,2,4-butantriol und 0,5 ml konz. Schwefelsäure in 1 l Aceton werden 258 ml 2,2-Dimeth-oxypropan innerhalb 2 Stunden getropft. Nach weiteren 4 Stunden ist die Umsetzung beendet. Durch Zugabe von 3,8 g Bariumhydroxid wird die Lösung neutralisiert. Es wird 30 Minuten nachgerührt, vom Feststoff abfiltriert und im Vakuum zur Trockne eingeengt. Das 2-Chlor-2-(2,2,dimethyl-1,3-dioxolan-4-yl)-ethanol wird als gelbes Öl erhalten.

Ausbeute: 340 g (94 % der Theorie).

3. 340 g 2-Chlor-2-(2,2-dimethyl-1,3-dioxolan-4-yl)-ethanol werden in 1,5 l abs. Äther gelöst. Bei 5°C werden unter intensivem Rühren insgesamt 130 g gepulvertes Kaliumhydroxid innerhalb 30 Minuten zugegeben, wobei die Temperatur durch Kühlung zwischen 5-15°C gehalten wird. Danach wird die Suspension bei 40°C 2 Stunden zum gelinden Rückfluss erhitzt. Nach dem Abstellen von Heizung und Rührer beginnen sich die Phasen zu trennen. Nach Stehen über Nacht wird über Kieselgur abgesaugt. Der Rückstand wird mit Äther extrahiert. Die vereinigten Ätherextrakte werden über eine 60 cm Vigreuxkolonne eingeengt. Der Rückstand (260 g) wird im Vakuum destilliert. Dabei destillieren 180 g einer farblosen Flüssigkeit über, die fraktioniert werden. Das 2-(2,2-Dimethyl-1,3-dioxolan-4-yl)-ethylenoxid wurde bei 65°C/13 mm erhalten.

Ausbeute: 154,5 g (57 % der Theorie).

Werden die Verbindungen der Formel I nach der Verfahrensvariante b) hergestellt, geht man von entsprechenden Azid der Formel III aus, das nach Methoden, die dem Fachmann bekannt sind, hydriert wird. So kann man beispielsweise die Azidverbindung III in einem geeigneten Lösungsmittel wie beispielsweise Methanol, Ethanol oder mit Wasserstoff in Gegenwart eines Edelmetallkatalysators wie beispielsweise

Palladium/Kohle in das Amin der Formel I überführen. Diese Umsetzung ist auch durchführbar, indem man den benötigten Wasserstorf in Gegenwart der Azidverbindung III erzeugt, beispielsweise durch Verwendung von Hydrazinhydrat und Raney-Nickel in einem geeigneten Lösungsmittel wie Methanol/Ethanol oder das Gemisch zum Sieden erhitzt und so das Amin der Formel I erhält.

Die für die Verfahrensvariante b) benötigten Ausgangsverbindungen der Formel III werden nach folgendem Reaktionsschema erhalten ($R^1$ und $R^2$ haben die oben angegebene Bedeutung):

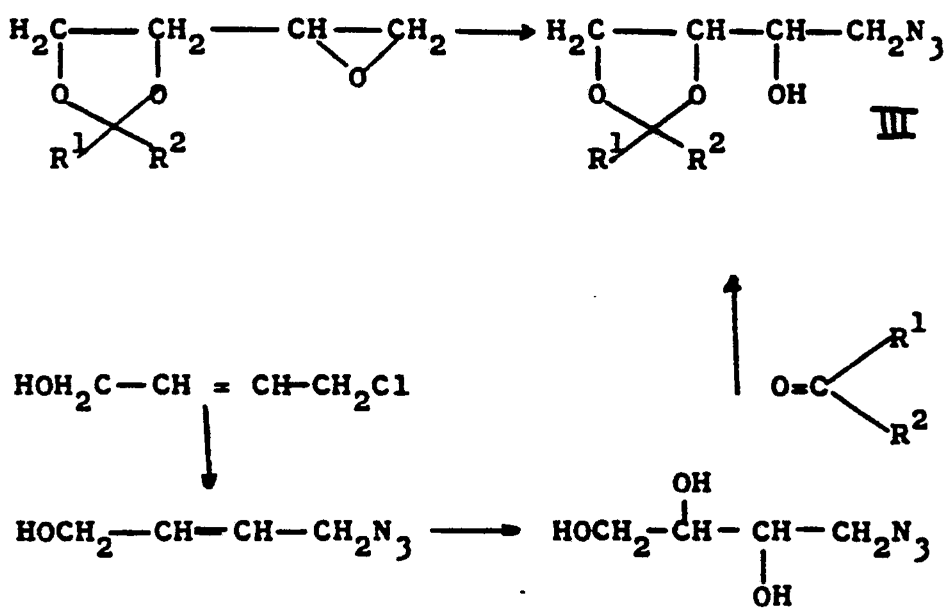

III

Die folgenden Herstellungsvorschriften sollen diese Reaktionen erläutern:

**2-Azido-(2,2-dimethvl-1,3-dioxolan-4-yl)-ethanol**

A. In einem Gemisch aus 50 ml Wasser und 100 ml Dioxan werden 4,23 g Natriumazid gelöst. Man erwärmt auf 60° C, tropft 8,65 g 2-(2,2-Dimethyl-1,3-dioxolan-4-yl)-ethylenoxid in 10 ml Dioxan zu und erwärmt 2 Stunden auf 80° C. Nach dem Abkühlen wird die Wasserphase mit Natriumchlorid gesättigt und die organische Phase abgetrennt, über Natriumsulfat getrocknet und im Vakuum zur Trockne eingeengt. Der Rückstand wird im Wasserstrahlvakuum destilliert. Das 2-Azido-(2,2-dimethyl-1,3-dioxolan-4-yl)-ethanol geht bei 130-32°/13mm (1710,5 Pa) als farbloses Öl über. Ausbeute: 9,1 g (81 % der Theorie).

B1. **4-Azido-but-2-en-ol**

Zu der 60° C warmen Lösung von 7,8 g Natriumazid in einem Gemisch aus 50 ml Wasser und 70 ml Dioxan werden unter Rühren innerhalb 20 Minuten 10,65 g 4-Chlor-but-2-en-ol getropft. Es wird 16 Stunden bei 60° C gerührt, im Vakuum zur Trockne eingeengt und im Vakuum destilliert. Das 4-Azido-but-2-en-ol geht als farbloses Öl bei 72-73/14mm (1842,1 Pa) über.

Ausbeute: 8,71g (77 % der Theorie).

B2. **4-Azido-1,2,3-butantriol**

In einer auf 70° C erwärmten Lösung von 11,3 g 4-Azido-but-2-en-ol in 90 ml Wasser wird unter gutem Rühren eine Lösung von 700 mg Wolframtrioxid in 11,3 ml Perhydrol zugetropft. Nach 3 Stunden lässt sich kein Peroxid mit angesäuerter Kaliumjodidlösung mehr nachweisen. Die Wolframsäure wird durch Zugabe von basischem Ionenaustauscher entfernt. Die Lösung wird im Vakuum zur Trockne eingeengt. Der Rückstand ist ein viskoses Öl. Ausbeute: 13,1 g (83 % der Theorie).

B3. **2-Azido-(2,2-dimethyl-1,2-dioxolan-4-yl)-ethanol**

Zu einer Lösung von 14,71 g 4-Azido-1,2,3-butan-triol in 30 ml Aceton werden 13,95 g 2,2-Dimeth-oxypropan sowie 0,1 ml konz. Schwefelsäure gegeben. Es wird über Nacht bei Raumtemperatur gerührt, mit 2N Natronlauge neutralisiert und im Vakuum zur Trockne eingeengt. Der Rückstand wird im Wasserstrahlvakuum destilliert. Das 2-Azido-(2,2-dimethyl-1,3-dioxolan-4-yl)-ethanol geht bei 130-32° C13 mm (1710,5 Pa) als farbloses Öl über.

Ausbeute: 11,79 g (63 % der Theorie).

Die nachfolgenden Beispiele sollen die Erfindung näher erläutern:

**Beispiel 1:**

Eine Lösung von 73,4 g 2-(2,2-Dimethyl-1,3-Dioxolan-4-yl)-ethylenoxid in 400 ml wässrigem Ammoniak (25 %ig) wurde 4 stunden auf 130°C im Autoklaven erhitzt. Die leicht gelbliche Lösung wurde im Vakuum zur Trockne eingeengt. Der Rückstand kristallisierte. Das 2-Amino-1( 2,2-dimethyl-1,3-dioxolan-4-yl)-ethanol wurde aus Ethanol/Ether umkristallisiert und zeigte einen Schmelzpunkt von 94 - 96° C
Ausbeute: 52,8 g (64,2 % der Theorie).

**Beispiel 2:**

Zu einer Lösung von 9,36 g 2-azido-1-(2,2-dimethyl-1,3-dioxolan-4-yl)-ethanol in 150 ml Methanol werden 900 mg Palladium/Kohle-Katalysator (10 %) gegeben. Es wird 1 Stunde bei Normaldruck hydriert. Dann wird vom Katalysator abfiltriert und mit Methanol nachgewaschen. Die vereinigten Lösungen werden im Vakuum zur Trockne eingeengt. Das verbleibende Öl wird aus Ethanol/Ether kristallisiert. Man erhält so 6,29 g (78 % der Theorie) 2-Amino-1-(2,2-di-methyl-1,3-dioxolan-4-yl)-ethanol in weissen Nadeln vom Schmelzpunkt 94-96° C.

**Beispiel 3:**

Zu einer Lösung von 9,36 g 2-Azido-1-(2,2-dimethyl-1,3-dioxolan-4-yl)-ethanol in 50 ml Ethanol werden 4,2 g Hydrazinhydrat und eine Spatelspitze Raney-Nickel (~ 200 mg) gegeben. Dann wird unter Rühren zum Sieden erhitzt, wobei eine sehr lebhafte Gasentwicklung einsetzt, die nach 20 Minuten beendet ist. Die abgekühlte Lösung wird durch Filtration vom Katalysator befreit und im Vakuum zur Trockne eingeengt. Der ölige Rückstand wird aus Ethanol/Ether kristallisiert. Das 2-Amino-1-(2,2-dimethyl-1,3-dioxolan-4-yl)-ethanol wird in weissen Nadeln erhalten.
Ausbeute: 6,69 g (83 % der Theorie), Schmelzpunkt 94-96° C.

**Patentansprüche**

1. 2-Amino-1-(1,3-dioxolan-4-yl)-ethanol-Verbindungen der allgemeinen Formel I

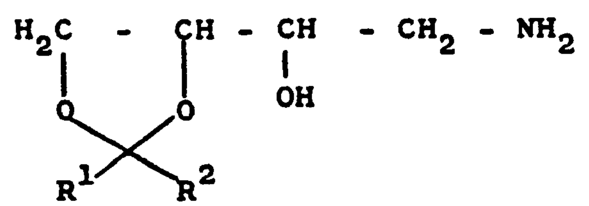

(I),

worin $R^1$ und $R^2$ gleich oder verschieden sind und Wasserstoff, eine niedere Alkylgruppe mit 1 bis 4 Kohlenstoffatomen darstellt oder, wenn $R^1$ eine Alkoxygruppe mit 1 bis 4 Kohlenstoffatomen bedeutet, $R^2$ eine Alkylgruppe mit 1 bis 4 Kohlenstoffatomen darstellt, oder $R^1$ und $R^2$ zusammen einen Alkylenrest mit 5 oder 6 Kohlenstoffatomen bedeuten.

2. 2-Amino-1-(2,2-dimethyl-1,3-dioxolan-4-yl)-ethanol.

3. Verfahren zur Herstellung von 2-Amino-1-(1,3-dioxolan-4-yl)-ethanol-Verbindungen der allgemeinen Formel I gemäss Anspruch 1, dadurch gekennzeichnet, dass man

a) ein 1,3-Dioxolanepoxid der allgemeinen Formel II

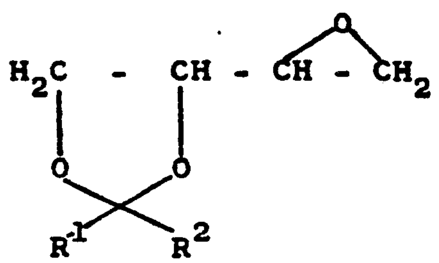

(II),

worin $R^1$ und $R^2$ die im Anspruch 1 angegebenen Bedeutungen besitzen, mit $NH_3$ umsetzt,
oder
b) eine Azid-Verbindung der allgemeinen Formel III

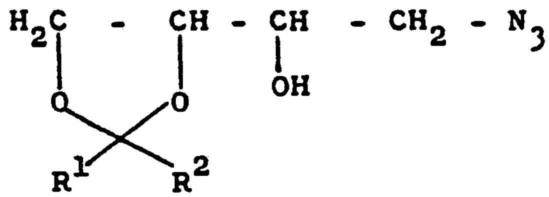

(III),

worin $R^1$ und $R^2$ die im Anspruch 1 angegebenen Bedeutungen besitzen, hydriert.

4. Verwendung von 2-Amino-1-(1,3-dioxolan-4-yl)-ethanol-Verbindungen gemäss Anspruch 1 bis 2 zur Herstellung von 5-[,N-(2-Hydroxyäthyl)-acetylamino]-2,4,6-trijod-isophthalsäure-bis-(2,3,4-trihydroxy-but-1-yl)-diamid (IV).

## Claims

1. 2-Amino-1-(1,3-dioxolan-4-yl)-ethanol compounds of the general formula

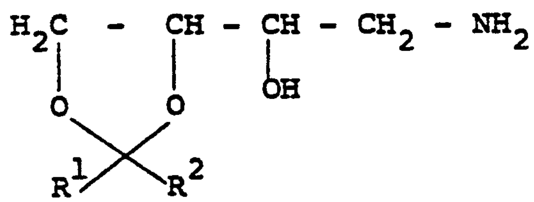

(I),

in which $R^1$ and $R^1$ are the same or different and represent hydrogen, a lower alkyl group having from 1 to 4 carbon atoms or, if $R^1$ represents an alkoxy group having from 1 to 4 carbon atoms, $R^2$ represents an alkyl group having from 1 to 4 carbon atoms, or $R^1$ and $R^2$ together represent an alkylene radical having 5 or 6 carbon atoms.

2. 2-Amino-1-(2,2-dimethyl-1,3-dioxolan-4-yl)-ethanol.

3. Process for manufacturing 2-amino-1-(1,3-dioxolan-4-yl)-ethanol compounds of the general formula 1 according to claim 1, characterised in that

per se

a) a 1,3-dioxolane epoxide of the general formula II

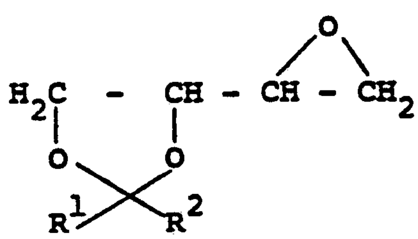

(II),

in which $R^1$ and $R^2$ have the meanings given in claim 1, is reacted with $NH_3$

or

b) an azide compound of the general formula III

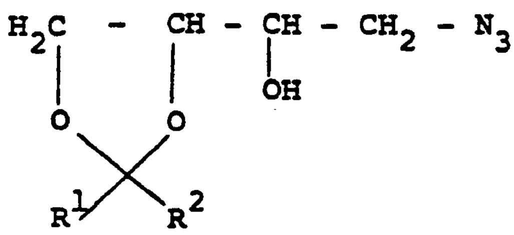

(III),

in which $R^1$ and $R^2$ have the meanings given in claim 1, is hydrogenated.

4. Use of 2-amino-1-(1,3-dioxolan-4-yl)-ethanol compounds according to claims 1 to 2 for manufacturing 5-[N-(2-hydroxyethyl)-acetamino]-2,4,5-triiodoisophthalic acid bis (2,3,4-trihydroxybut-1-yl)-diamide (IV).

**Revendications**

1. Amino-2 (dioxolanne-1,3 yl-4)-1 éthanols répondant à la formule générale I:

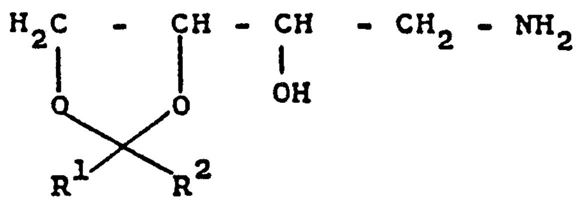

(I)

dans laquelle :

ou bien $R^1$ et $R^2$ représentent chacun, indépendamment l'un de l'autre, l'hydrogène ou un radical alkyle inférieur contenant de 1 à 4 atomes de carbone,

ou bien $R^1$ représente un radical alcoxy contenant de 1 à 4 atomes de carbone et $R^2$ un radical alkyle contenant de 1 à 4 atomes de carbone,

ou bien $R^1$ et $R^2$ forment ensemble un radical alkylène contenant 5 ou 6 atomes de carbone.

2. Amino-2 (diméthyl-2,2 dioxolanne-1,3 yl-4)-1 éthanol.

3. Procédé de préparation d'amino-2 (dioxo-lanne-1,3 yl-4)-1 éthanols de formule générale 1 selon la revendication 1, procédé caractérisé en ce que:

a) on fait réagir avec $NH_3$ un époxy-dioxo-lanne-1,3 répondant à la formule générale II:

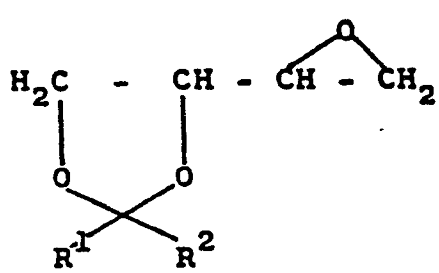

(II)

dans laquelle $R^1$ et $R^2$ ont les significations données à la revendication I, ou
b) on hydrogène un azide répondant à la formule générale III:

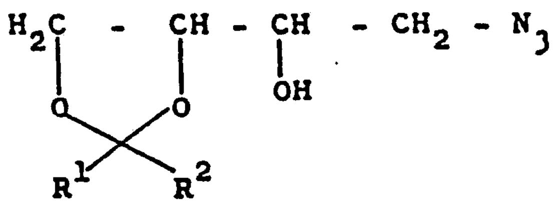

(III)

dans laquelle $R^1$ et $R^2$ ont les significations données à la revendication I.

4. Application d'amino-2 (dioxolanne-1,3 yl-4)-1 éthanols selon l'une des revendications 1 et 2 pour la préparation du N,N'-bis-(trihydroxy-2,3,4 butyl)-diamide de l'acide (N-(hydroxy-2 éthyl)-N-acétyl-amino)-5 triodo-2,4,6 isophtalique (IV).